# EUROPEAN PATENT APPLICATION

(11) **EP 1 914 318 A1**
(43) Date of publication of application: **23.04.2008**
(21) Application number: 06782598.4
(22) Date of filing: 10.08.2006
(51) Int. Cl.: C12Q 1/68, C12N 15/09

(54) **METHOD OF DETECTING LARGE BOWEL CANCER MARKER**

(30) Priority: 10.08.2005 JP 2005231972
(71) Applicant: Hamamatsu Foundation for Science and Technology Promotion, Hamamatsu-shi, Shizuoka 432-8011 (JP)
(72) Inventor: KANAOKA, Shigeru, (JP)
(74) Representative: Becker, Philippe
(86) International application number: PCT/JP2006/315793
(87) International publication number: WO 2007/018257

(57) **Abstract**

A method of detecting a tumor marker for the diagnosis of large bowel cancer and adenomatous polyposis coli comprising the steps of a) collecting and freezing feces, b) homogenizing the frozen feces in the presence of an RNAase inhibitor and preparing a suspension, c) extracting RNA from the obtained sample from which RNA is extracted, d) obtaining cDNA by reverse transcribing the extracted RNA, e) amplifying the obtained cDNA, and f) detecting the amplified cDNA, **characterized in that** the tumor marker is one or more tumor markers selected from the group consisting of COX-2, SNAIL and MMP-7 (with the proviso that a case where only COX-2 or MMP-7 is selected is excluded).

## Description

### [Field of the invention]

The present invention relates to methods for detecting one or more tumor markers selected from the group consisting of COX-2, SNAIL and MMP-7 (except for COX-2 alone and MMP-7 alone) for diagnosis of colon cancer and colon adenoma, and to diagnostic kits for colon cancer and colon adenoma comprising these tumor markers.

### [Background of the invention]

Death toll from the colon cancer has been increasing. The death toll from the colon cancer is the 4th largest in men and the 2nd largest in women among the toll from all the cancers (the cancer death statistics for 1999 in Japan). In the cancer patient estimation in 2015, the colon cancer death toll is estimated to be the 1st largest in both men and women, and thus synthetic counterplan against the colon cancer including secondary prevention is demanded. Mass screening of the cancer is one of the most effective methods.

For the mass screening of the cancer, it is important to adopt a simple and noninvasive detecting method. The only noninvasive method available now is the examination of occult-blood in feces, i.e., fecal occult blood test, which is widely used as the standard method of the mass screening of the colon, cancer.

However, since presence of hemoglobin in the feces is not specific for the tumor, the fecal occult blood test has low sensitivity and specificity (the sensitivity 30 - 90%, the specificity 70 - 98%) and has a disadvantage that not a little false negative and false positive exist.

Also, in the diagnosis of colon cancer, entire colon endoscopy or combination of barium enema inspection and sigmoid colon, endoscopy has been carried out, at the same time with or after the screening by the immunochemical fecal occult blood method, and thus there has been a disadvantage of consuming great time and effort.

As alternative methods to the fecal occult blood test, methods utilizing DNA in the feces that detect K-ras, p-53 or APC gene mutation, microsatellite instability or the like have been reported (Nonpatent documents 1-4).

These methods utilizing DNA have the characteristics that they are non-invasive methods possible to catch direct changes of cancer cells and have high specificity, and thus they are considered to be a highly potent method. But they have the disadvantages that they have low sensitivity compared to the conventional fecal occult blood test and also are rather time and effort consuming.

As further alternative methods to the fecal occult blood test, methods of detecting mRNA, such as protein kinase C (PKC) mRNA, in the feces have also been developed, in order to detect the genetic expression more directly (Nonpatent documents 5-7).

However, with the above-mentioned methods using RNA, RNA could not be extracted from small amount of feces simply and efficiently, and the sensitivity exceeding the fecal occult blood method could not be obtained.

A qualitative and quantitative RNA detection method is known that combines the PCR technique with the reverse transcriptase reaction (RT). This RT-PCR method is superior to the Northern blotting method in high sensitivity capable of detecting trace molecules, and is superior to the in situ hybridization method in speed and ease of the technique.

However, since RNA is more unstable compared to DNA, and is always exposed to the danger of degradation by very stable RNases present universally in all the biological samples, strict management is required in order not to be contaminated by RNases in the process of the RT-PCR method and during and after purification process of RNA.

Accordingly, when extracting RNA from feces which are biologically very crude samples, the step of pre-separating the cell fraction has been required, in order to eliminate the influence of RNases.

Therefore, it has been thought impossible to detect RNA directly in the feces in which a huge amount of RNases originating from a lot of microorganisms are present, and at least separation of the cell fraction has been thought indispensable for elimination, of exogenous RNases originating from the microorganisms and the like.

The present inventor has already developed a method for detecting useful tumor markers in the feces to diagnose the colon cancer, by homogenizing optionally frozen biological samples in the presence of RNase inhibitors (Patent document 1).

As an alternative colon cancer screening procedure to the fecal occult blood method, a method to investigate the expression of the CD44 variant in the cell fraction of the feces has been recently developed, but its sensitivity was only about 68% which was far inferior to the sensitivity of the fecal occult blood method (about 75%) (Nonpatent document 8).

The present inventor has surprisingly found that by adopting one or more tumor markers selected from the group consisting of COX-2, SNAIL and MMP-7 (except for COX-2 alone and MMP-7 alone), sensitivity, specificity and accuracy of the diagnosis of the colon cancer and the colon adenoma could be improved, and has completed the present invention.
Patent document 1: WO2004/083856A1
Nonpatent document 1: D. Sidransky, et al., Science, vol. 256, Apr. 3, 1992, pp.102 - 105
Nonpatent document 2: S. M. Dong, et al., Journal of the National Cancer Institute, vol. 93, No.11, June 11, 2001, pp.858 - 865.
Nonpatent document 3: G. Traverso, et al., The New England Journal of Medicine, vol.346, No. 5, Jan. 31, 2002, pp. 311-320.
Nonpatent document 4: G. Traverso, et al., The Lancet, vol. 359, Feb. 2, 2002, pp.403 - 404.
Nonpatent document 5: L. A. Davidson, et al., Carcinogenesis, vol. 19, No. 2, 1998, pp. 253 - 257. Nonpatent document 6: R.J. Alexander and R.F. Raicht, Digestive Diseases and Sciences, vol.43, No. 12, 1998, pp. 2652 - 2658.
Nonpatent-document 7: T.Yamao et al., Gastroenterology, vol. 114, No. 6, 1998, pp. 1198 - 1205.
Nonpatent document 8: Hiroshi Saito, Japanese Journal of Cancer Research, vol. 87, No. 10, 1996, pp. 1011 - 1024.

### SUMMARY OF THE INVENTION

### Problems to be solved by the invention

Therefore, the object of the present invention resides in providing methods for detecting one or more tumor markers selected from the group consisting of COX-2, SNAIL and MMP-7 (except for COX-2 alone and MMP-7 alone) for diagnosing colon cancer and colon adenoma, and in proving a diagnostic kit for diagnosing colon cancer and colon adenoma comprising these tumor markers.

More specifically, the present invention is related to the followings:
1. A method for detecting tumor markers for diagnosing colon cancer and colon adenoma, comprising the following steps:
   a) sampling and freezing feces,
   b) homogenizing the frozen feces in the presence of RNase inhibitors to prepare a suspension,
   c) extracting RNA from the obtained sample for extracting the RNA,
   d) subjecting the extracted RNA to reverse transcription to give cDNA,
   e) amplifying the obtained cDNA, and
   f) detecting the amplified cDNA,
   characterized in that said tumor markers are one or more tumor markers selected from the group consisting of COX-2, SNAIL and MMP-7 (except for COX-2 alone and MMP-7 alone).
2. The method described in the above 1, wherein said tumor markers are a combination of COX-2 and MMP-7, a combination of COX-2, SNAIL and MMP-7 or a combination of COX-2 and SNAIL.
3. The method described in the above 1 for diagnosing colon cancer, wherein said tumor markers are a combination of COX-2, SNAIL and MMP-7, and the colon cancer is an early stage colon cancer.
4. A kit for detecting tumor markers for diagnosing colon cancer and colon adenoma, comprising the following means:
   a) means for sampling and freezing feces,
   b) means for homogenizing the frozen feces in the presence of RNase inhibitors to prepare a suspension,
   c) means for extracting RNA from the obtained sample for extracting the RNA,
   d) means for subjecting the extracted RNA to reverse transcription to give cDNA,
   e) means for amplifying the obtained cDNA, and
   f) means for detecting the amplified cDNA,
   characterized in that said tumor markers are one or more tumor markers selected from the group consisting of COX-2, SNAIL and MMP-7 (except for COX-2 alone and MMP-7 alone).
5. The kit for detecting tumor markers for diagnosing colon cancer and colon adenoma described in the above 4, wherein said tumor markers are a combination of COX-2 and MMP-7, a combination of COX-2, SNAIL and MMP-7, or a combination of COX-2 and SNAIL.
6. The kit for detecting tumor markers for diagnosing colon cancer and colon adenoma described in the above 4, wherein said tumor markers are a combination of COX-2, SNAIL and MMP-7, and the colon cancer is an early stage colon cancer.

### BEST MODE FOR CARRYING OUT THE INVENTION

The RNase inhibitors according to the present invention include guanidine thiocyanate, Isogene, Ultraspec II (Registered Trademark) and the like.

As the freezing method, any conventional technologies, preferably methods using liquid nitrogen can be used. The freezing temperature and the storage temperature are -1 to -196°C, preferably -20 to -196°C, preferably -75 to -196 °C, more preferably -110 to -196 °C, and most preferably - 196°C.

The frozen sample may be frozen stored. The storage temperature is -75 to -196 °C, preferably -110 to -196 °C, and more preferably -196 °C. The storage period is 1 day - 10 years, preferably 1 day - 3 years, and more preferably 1 day - 1 year.

The tumor markers used in the present invention are one or more tumor markers selected from the group consisting of COX-2, SNAIL and MMP-7 (except for COX-2 alone and MMP-7 alone).

Preferably, above-mentioned tumor markers are a combination of COX-2 and MMP-7, a combination of COX-2, SNAIL and MMP-7 or a combination of COX-2 and SNAIL.

For the diagnosis of early colon cancer and colon adenoma, the above-mentioned tumor markers are a combination of COX-2, SNAIL and MMP-7.

COX-2 is an inducible enzyme which converts arachidonic acid into prostaglandin H2, and if the expression of COX-2 increases, apoptosis will be inhibited, and angiogenesis factors and matrix degrading enzymes needed at the time of infiltration will be induced. SNAIL is a negative transcription regulatory factor of E-cadherin, an intercellular adhesion factor, and it is known that if the expression of SNAIL increases, expression of E-cadherin reduces and adhesion between cells decreases, influencing deep infiltration and metastasis. MMP-7 is a kind of matrix metalloproteinase, and is thought to be required for infiltration and metastasis of the cancer.

The above-mentioned steps d) to f) are called the RT-PCR method, and can be carried out according to the description by Takao Sekiya et al., eds., "The PCR Method Front Line", 1997, Kyoritsu Shuppan, pp.187 - 196, for example.

The extraction of RNA from a suspension can be carried out using conventionally known methods and, for example, commercial kits such as RNeasy Mini Kit (QIAGEN) or RNA Extraction Kit (Pharmacia Biotech) can be used.

Reverse transcription in the present invention refers to conversion of RNA into complementary DNA (cDNA) using the reverse transcriptase. The reverse transcription reaction is usually carried out using a solution containing a buffer, salts such as MgCl₂ and KCl, dithiothreitol (DTT), primers, deoxyribonucleotides, RNase inhibitors and the reverse transcriptase. The above-mentioned salts can be changed to other suitable salts. Proteins such as gelatin and albumin, surfactants and the like can also be added.

Amplification of cDNA following the reverse transcription is usually carried out by PCR. The PCR reaction mixture usually contains a buffer, salts such as MgCl₂ and KCl, primers, deoxyribonucleotides and a heat-resistant polymerase. The above-mentioned salts can be changed to other suitable salts. Proteins such as gelatin and albumin, dimethylsulfoxide, surfactants and the like can also be added.

Amplification of cDNA can also be carried out by the LAMP method (Japanese Patent Publication No. 3,313,358) and the ICAN method (Japanese Patent Application Publication, 2001-136965).

Primers in the present invention mean oligonucleotides which work as synthesis initiation sites in the cDNA synthesis or the nucleic acid amplification. Single strands are desirable for the primers, but double strands can also be used. When the primers are double strands, they are desirably converted into single strands in advance of the amplification reaction. The primers can be synthesized according to known methods, and can also be isolated from living organisms.

A reverse transcriptase used in the reverse transcription reaction means the enzyme that can reverse transcribe RNA into cDNA. The reverse transcriptases include reverse transcriptases derived from retroviruses such as RAV (Rous associated virus) and AMV (Avian myeloblastosis virus), and reverse transcriptases derived from mouse retroviruses such as MMLV (Moloney murine leukemia virus), but are not limited to these.

Heat-resistant polymerases used for PCR include Taq polymerase, but are not limited to this.

Electrophoresis utilizing agarose gel can be used as a detection method of the amplified DNA without limitation.

The kit according to the present invention can also include instruction describing the method of the present invention. The contents of the kit include a reverse transcriptase, RNase inhibitors, a DNA synthetase, various kinds of deoxynucleotide triphosphates (dNTPs), primers specific to each of the tumor markers, positive control to each of the tumor markers, and distilled water not containing RNases or DNases, reagents for visual fluorescence detection, and the like.

### Example 1

Following Example illustrates the present invention, but never limits the present invention.

Patients hospitalized for inspection and treatment in First Department of Medicine, Hamamatsu University School of Medicine who were identified to have the colon cancer by the total colonoscopy, and patients who had no tumor or inflammatory change in the colon (non-colon disease patients) were examined. Informed consents were obtained from all the patients.

Feces were dispensed in aliquots of about 0.5 - 1 g into 5 ml tubes as soon as possible after sampling feces, frozen by liquid nitrogen, and stored at -80°C. Human hemoglobin (Hb) in each sample feces was measured by the immunochemical fecal occult blood test for comparison. The biopsy specimens from cancer regions and normal regions of the tissue obtained at the time of the endoscopy before treatment were frozen with liquid nitrogen and were stored at -80 °C. Then, they were homogenized using a homogenizer, guanidine salt and phenol, and total RNA was extracted by chloroform and ethanol.

1µg of the obtained RNA was subjected to reverse transcription using ReverScript II (Registered trademark) (reaction mixture 20µl, Wako Pure Chem.) to give cDNA. A part thereof was amplified by nested PCR using GeneTaq (Wako Pure Chem.). The obtained PCR amplification product was electrophoresed on a 4% agarose gel, and was stained with ethidium bromide.

Primers used were random primers in the reverse transcription, and were originally designed primers in the PCR for all of COX-2, SNAIL and MMP-7. In the PCR, the 1st round consisted of 20 cycles for each of the three molecules, and the 2nd round consisted of 20 cycles for COX-2 and 35 cycles for both SNAIL and MMP-7. The used primers are shown below:
<COX-2>
   Forward 1. : 5'-CTGAAAACTCCAAACACAG-3'
   Forward 2 : 5'-GCACTACATACTTACCCACTTCAA-3'
   Reverse: 5'-ATAGGAGAGGTTAGAGAAGGCT-3'
<SNAIL>
   Forward 1 : 5'-AGATGAGGACAGTGGGAAAGGCT-3
   Forward 2 : 5'-CCTTCGTCCTTCTCCTCTACTTCA-3'
   Reverse 1 : 5'-AGGTATGGAGAGGAAGAGGGAG-3'
   Reverse 2 : 5'-GCACTGGTACTTCTTGACATCTGAG-3'
<MMP-7>
   Forward 1 : 5-ATGAGTGAGCTACAGTGGGAA-3'
   Forward 2 : 5'-TTAAACTCCCGCGTCATAGAA-3'
   Reverse 1 : 5-AAATGCAGGGGGATCTCTTTG-3'
   Reverse 2 : 5-TCGATCCACTGTAATATGCGG-3'

### Results

The results of detection by RT-PCR of COX-2, SNAIL and MMP-7 in the feces from 65 colon cancer patients (13 early cancer patients, 52 advanced cancer patients) and from 32 patients in the control group, were compared with the detection results of the immunological fecal occult blood test conducted for comparison (Table 1).

| Comparison of the COX-2, SNAIL and MMP-7 assay with the fecal occult blood method | | | | | | | |
|---|---|---|---|---|---|---|---|
| Tumor marker | COX-2 | SNAIL | MMP-7 | COX-2 or SNAIL | COX-2 or MMP-7 | COX-2, SNAIL or MMP-7 | Immunological fecal occult blood method |
| Sensitivity 95% Cl | 87.7% (57/65) 77.2 - 94.5% | 49.2% (32/85) 36.6-61.9% | 65.6% (42/64) 52.7-77.1% | 87.7% (57/65) 77.2-94.5% | 90.8% (59/65) 81.0-96.5% *P* = *0,02* | 90.8% (59/65) 81.0-96.5% *P*=0.02 | 73.0% (46 /63) 60.3-83.4% |
| Specificity 95% Cl | 100% (32/32) 91.1-100% | 100% (32/32) 91.1-100% | 100% (31 / 31) 90.8-100% | 100% (32/32) 91.1-100% | 100% (31/31) 90.8-100% | 100% (31/31) 90.8-100% | 87.5% (28/32) 71.0-96.5% |
| Accuracy 95% Cl | 91.7% (89/97) 84.4-96.4% *P* = *0.01* | 66.0% (64/37) 55.7-75.3% | 76.8% (73 / 95) 67.1-84.9% | 91.7% (89/97) 84.4-96.4% *P=0.01* | 93.8% (90/96) 86.9-97.7%* *P* = *0.003* | 93.8% (90/96) 86.9-97.7%* *P*=0,003 | 77.9% (74/95) 68.2-85.8% |
| Adenoma | 61.5% (8/13) 31.6-86.1% | 61.5% (8/13) 31.6-66.1% | 53.8% (7/13) 25.1-74.1% | 76.9% (10/13) 46.2-95.0% *P* = 0.049 | 69.2% (9/13) 38.6-90.9% | 34.6%(11/13) 54.6-98.1% *P*=0.017 | 30.8% (4/13) 9.1-61.4% |
| Adenotia ~ Stage 0 cancer | 66.7% (10/15) 38,4-88,2% | 53.3% (8/15) 26.6-78.7% | 46.7% (7/15) 21.3-73.4% | 80.0% (12/15) 51.3-95.7% *P*=0.010 | 73.3% (11/15) 44.9-92.2% *P*=0.028 | 86.7%(13/15) 59.5-98.3% *P*=0.0032 | 26.7% (4/15) 7.8-55.1% |
| Adenoma ~ Stage I cancer | 73.1% (19/26) 52.2-88.4% *P = 0.017* | 50.0% (13/26) 29.9-70.1% | 50.0% (13 /26) 29.9-70.1% | 80,8% (21/26) 60.6-93.4%* *P = 0,0030* | 76.9% (20/26) 56.4-91.0% *P =0.0076* | 34.6% (22/26) 85.1-95.6%* *P* = 0.0010 | 36.0% (9/25) 18.0-57.5% |
| Adenoma ~ Stage II cancer | 83.6% (46/55) 71.2-92.2% *P=0.013* | 47.3% (26/55) 33.7-61.2% | 64.8% (35 /54) 50.6-77.3% | 87.3% (48/55) 75.5-94.7%** P = 0.0029* | 87.3% (48/55) 75.5-94.7% *P = 0.0029* | 90.9% (50/55) 80.0-97.0%* *P*=0.00048 | 60.4% (32/53) 46.0-73.5% |
| Adenoma and entire cancer | 83.3% (65/78) 73.1-90.8% *P* = *0.020* | 48.7% (38/78) 37.2-60.3% | 63.6% (49 / 77) 51.9-74.3% | 85.9% (67/78) 76.2-92.7% *P* = *0.0063* | 87.2% (68/78) 77.7-93.7% *P = 0.0032* | 89.7% (70/78) 80.8-95.4%* *P* = 0.00070 | 65.8 (50/76) 54.0-76.3% |
| Accuracy for adenoma, entire cancer and normal person | 88.2% (97/110) 80.6-93.6%* *P* = *0.0053* | 70.9% (78/110) 61.5-79.2% | 80.6% (87/108) 71.8-87.5% | 90,0% (99/110) 82.8-94.9% * *P = 0.0014* | 90.8% (99/109) 83.8-95.5%* *P* = 0.00078 | 92.7% (101/109) 86.0-96.8%* *P* = 0.00016 | 72.2(78/108) 62.8-80.4% |

The sensitivity, specificity, and accuracy (percent ability to diagnose cancer as cancer and normal as normal) for 65 colon cancer patients were: 87.7% (57/65), 100% (32/32) and 91.7% (89/97) respectively for COX-2; 49.2% (32/65), 100% (32/32) and 66.0% (64/97) respectively for SNAIL; and 65.6% (42/64), 100% (31/31) and 76.8% (73/95) respectively for MMP-7.

On the other hand, in the immunochemical fecal occult blood test conducted for comparison, sensitivity was 73.0% (46/63), specificity 87.5% (28/32) and accuracy 77.9% (74/95).

SNAIL alone or MMP-7 alone had sensitivity and accuracy with lower values compared with the immunochemical fecal occult blood test. But COX-2 had higher values for any of the parameters: sensitivity, specificity and accuracy, than immunochemical fecal occult blood test. However, as shown in Table 1, there were no significant statistical differences: 95% confidence intervals (95% CI) of all the parameters for COX-2 overlapped with those for the immunochemical fecal occult blood test.

Here, since MMP-7 was positive in two examples among eight COX-2 negative examples, the combination of two markers, COX-2 and MMP-7, was examined. That is, if samples with either COX-2 or MMP-7 positive were grouped as a positive group (COX-2 or MMP-7), the sensitivity was 90.8% (59/65) and its accuracy was 93.8% (90/96). As a result, the sensitivity and the accuracy of the COX-2 or MMP-7 group were statistically different from those for the immunochemical fecal occult blood test. The 95% confidence interval of its accuracy was 86.9% - 97.7%, and that of the immunochemical fecal occult blood test was 68.2% - 85.8%. As a result there was no overlapping. Similarly, when the samples with either COX-2, SNAIL or MMP-7 positive were grouped (COX-2, SNAIL or MMP-7), it was shown that the sensitivity of this group was also significantly different from that of the immunochemical fecal occult blood test. Also the accuracies of the groups: COX-2 ; COX-2 or SNAIL; COX-2 or MMP-7; and COX-2, SNAIL or MMP-7, were statistically significantly different from that of the immunochemical fecal occult blood test.

Next, cancer was classified into the clinical stages 0 to IV, and the clinical cases were classified into four groups: adenoma, adenoma to stage 0 cancer, adenoma to stage I cancer (adenoma, stage 0 cancer and stage I cancer), adenoma to stage II cancer (adenoma, and cancer from stage 0 to stage II), and adenoma and the entire cancer (advanced neoplasia including adenoma and cancer), and the sensitivity was examined in each group.

The result was that even COX-2 alone had the sensitivities of 73.1%, 83.6%, and 83.3% respectively for adenoma to stage I cancer, adenoma to stage II cancer, and adenoma and the entire cancer, while the immunochemical fecal occult blood test had the sensitivities of 36.0%, 60.4% and 65.8% for each of the same groups. Also it had the accuracy of 88.2% (97/110) and its 95% confidence interval was 80.6% - 93.6%, while the accuracy for the immunochemical fecal occult blood test was 72.2% (78/108) and the 95% confidence interval was 62.8% - 80.4%.

That is to say, it was shown that even COX-2 alone had the sensitivity and the accuracy statistically significantly exceeding those of the immunochemical fecal occult blood test, in each of the groups: adenoma to stage I cancer, adenoma to stage II cancer, and adenoma and the entire cancer, and the 95% confidence intervals of the two did not overlap statistically.

Among five COX-2 negative adenoma examples, one was MMP-7 positive and two were SNAIL positive. Thus, such cases were also examined, where patients with COX-2 or MMP-7 positive; COX-2 or SNAIL positive; and COX-2, SNAIL or MMP-7 positive were grouped as a positive group, respectively.

As a result, for the adenoma only, the sensitivity of COX-2 or SNAIL was 76.9% (10/13, the 95% confidence interval 46.2% - 95.0%), and the sensitivity of COX-2, SNAIL or MMP-7 was 84.6% (11/13, the 95% confidence interval 54.6% - 98.1%). Both were far higher than the sensitivity of the immunochemical fecal occult blood test of 30.8% (4/13, the 95% confidence interval 9.1% - 61.4%). In addition, for: adenoma to stage 0 cancer; adenoma to stage I cancer; adenoma to stage II cancer; and adenoma and the entire cancer, the sensitivities of COX-2 or SNAIL positive, of COX-2 or MMP-7 positive, and of COX-2, SNAIL or MMP-7 positive, were statistically significantly different from that of the immunochemical fecal occult blood test. Furthermore, significantly higher sensitivities with no overlapping of the 95% confidence intervals could be obtained, in COX-2 or MMP-7 for adenoma to stage II cancer, in COX-2 or SNAIL for adenoma to stage I cancer, and in COX-2, SNAIL or MMP-7 positive for adenoma to stage 0 cancer.

In conclusion, the combination of COX-2, SNAIL and MMP-7 is excellent in screening of comparatively early colon tumors curable by endoscope or operation and the like, and is a very useful screening method having high specificity.

### Industrial applicability

The method of the present invention is a novel non-invasive screening method of the colon cancer with high specificity, sensitivity and accuracy, and thus can be an alternative of the conventional fecal occult blood method and is clinically very useful.

## Claims

1. A method for detecting tumor markers for diagnosing colon cancer and colon adenoma, comprising the following steps:
a) sampling and freezing feces,
b) homogenizing the frozen feces in the presence of RNase inhibitors to prepare a suspension,
c) extracting RNA from the obtained sample for extracting the RNA,
d) subjecting the extracted RNA to reverse transcription to give cDNA,
e) amplifying the obtained cDNA, and
f) detecting the amplified cDNA,
**characterized in that** said tumor markers are one or more tumor markers selected from the group consisting of COX-2, SNAIL and MMP-7 (except for COX-2 alone and MMP-7 alone).

2. The method according to claim 1, wherein said tumor markers are a combination of COX-2 and MMP-7, a combination of COX-2, SNAIL and MMP-7 or a combination of COX-2 and SNAIL.

3. The method according to claim 1, wherein said tumor markers are a combination of COX-2, SNAIL and MMP-7, and the colon cancer is an early stage colon cancer.

4. A kit for detecting tumor markers for diagnosing colon cancer and colon adenoma, comprising the following means:
a) means for sampling and freezing feces,
b) means for homogenizing the frozen feces in the presence of RNase inhibitors to prepare a suspension,
c) means for extracting RNA from the obtained sample for extracting the RNA,
d) means for subjecting the extracted RNA to reverse transcription to give cDNA,
e) means for amplifying the obtained cDNA, and
f) means for detecting the amplified cDNA,
**characterized in that** said tumor markers are one or more tumor markers selected from the group consisting of COX-2, SNAIL and MMP-7 (except for COX-2 alone and MMP-7 alone).

5. The kit for detecting tumor markers for diagnosing colon cancer and colon adenoma according to claim 4, wherein said tumor markers are a combination of COX-2 and MMP-7, a combination of COX-2, SNAIL and MMP-7 or a combination of COX-2 and SNAIL.

6. The kit for detecting tumor markers for diagnosing colon cancer and colon adenoma according to claim 4, wherein said tumor markers are a combination of COX-2, SNAIL and MMP-7, and the colon cancer is an early stage colon cancer.
